# EUROPEAN PATENT SPECIFICATION

(11) **EP 3 858 973 B1**
(45) Date of publication and mention of the grant of the patent: **21.09.2022**
(21) Application number: 19916522.6
(22) Date of filing: 06.05.2019
(51) Int. Cl.: C12M 1/38, C12M 1/36, C12M 1/34, C12M 1/12, C12M 1/02

(54) **SIMULATION SYSTEM FOR SIMULATING EXTREME ENVIRONMENT OF DEEP SEA HYDROTHERMAL VENT**
SIMULATIONSSYSTEM ZUR SIMULATION DER EXTREMEN UMGEBUNG VON HYDROTHERMALEN TIEFSEEENTLÜFTUNGEN
SYSTÈME DE SIMULATION POUR SIMULER UN ENVIRONNEMENT EXTRÊME D'UN ÉVENT HYDROTHERMIQUE EN MER PROFONDE

(30) Priority: 22.02.2019 CN 201910131901
(43) Date of publication of application: 04.08.2021
(73) Proprietor: China Ship Scientific Research Center (the 702 Institute of China Shipbuilding Industry Corporation), Wuxi, Jiangsu 214082 (CN)
(72) Inventor: LIU, Hao, Wuxi, Jiangsu 214082 (CN); YE, Cong, Wuxi, Jiangsu 214082 (CN)
(74) Representative: Murgitroyd & Company
(86) International application number: PCT/CN2019/085666
(87) International publication number: WO 2020/168638

(56) References cited:
- WO-A2-2009/004589
- CN-A- 103 540 522
- CN-A- 106 111 218
- CN-A- 106 706 263
- CN-A- 108 760 720
- CN-U- 206 972 628
- JP-A- H06 213 147
- DONG, Xianxin et al.: "A Design of Deep-Sea Extreme Environment Simulator", Journal of Tropical Oceanography, vol. 33, no. 2, 31 March 2014 (2014-03-31) , pages 101-108, XP055801275, DOI: 20191105153039
- PARK, C. B. et al.: "Rupture of the Cell Envelope by Decompression of the Deep-Sea Methanogen Methanococcus Jannaschii", Applied and Environmental Microbiology, vol. 68, no. 3, 31 March 2002 (2002-03-31) , pages 1458-1463, XP055728694, DOI: 20191105153414A

## Description

### TECHNICAL FIELD

The present invention relates to the technical field of microbiological culture, and particularly relates to a deep-sea hydrothermal vent extreme environment simulation system.

### BACKGROUND

The scalding hydrothermal fluid with a temperature of more than three hundred DEG C is sprayed upward from the submarine hydrothermal vent. When encountering icy seawater, the hydrothermal fluid will be mineralized into chimney-like metal sulfides with a height of 13 m. There are also a large number of seabed creatures near the chimney-like metal sulfides. These microorganisms have a series of characteristics including thermophilic, pressure-resistant or barophilic and antitoxic properties. Because the microorganisms are in the extreme hydrothermal environment of the deep sea, the samples obtained through scientific research ships are very limited, and these thermophilic or barophilic microorganisms are prone to death due to changes in environmental conditions.

Patent specification WO2009/004589A2 discloses a system allowing the study and preservation of aquatic organisms (mainly sea organisms) that inhabit deep depths, simulating the respective hydraulic pressures and allowing the controls of the necessary environmental conditions to the maintenance and development of the said organisms. Patent specification CN106706263A discloses a submarine hydrothermal exhalation simulation device having an observation function, mainly comprising a vertical high pressure injection pump, a temperature control system, a pressure regulating valve, a transparent pressure vessel, a high speed camera, a water cooling system, a pressure gauge, a sulfide collecting device, a pressure release valve and a stop valve.

### SUMMARY

### Technical Problems

Therefore, it is necessary to establish a deep-sea hydrothermal vent extreme environment simulation system in the laboratory to cultivate and study the microorganisms near the hydrothermal vent in an artificially simulated deep-sea hydrothermal environment. In this way, it is possible to conduct in-depth research on submarine hydrothermal eruption and surrounding unique life forms, thereby providing an important window for humans to study the origin of life and the deep-sea biosphere. In view of the above-mentioned existing problems, the applicant has conducted research improvement to provide a deep-sea hydrothermal vent extreme environment simulation system. The present invention can provide a hydrothermal vent simulation environment with a maximum temperature of 400°C and a maximum pressure of 250 MPa for deep-sea microorganisms, which can maintain the flow of a nutrient solution within the system at 0.1-10 ml/min, and the system can work continuously for one year under this condition.

### Solutions

### Technical Solutions

A deep-sea hydrothermal vent extreme environment simulation system for the culture of microorganisms includes an industrial personal computer, motor controllers of a plurality of water pumps are connected in parallel to the industrial personal computer, the inlet of each water pump is connected to a nutrient solution container, the outlet of each water pump is respectively connected to a pressure sensor and a non-return valve, the electric signal end of each pressure sensor is connected to the industrial personal computer, the outlet of each non-return valve is collected through a pipeline, the outlet collected by the pipeline is connected to the inlet of a cut-off valve, the outlet of the cut-off valve is connected to one end of a first hard tube, the other end of the first hard tube extends into a reaction kettle and is immersed at the bottom of the solution inside the kettle, the reaction kettle is arranged inside a sand bath pool, and an electric signal control port of the sand bath pool is also connected to the industrial personal computer. The system further includes a second hard tube connected to the reaction kettle, the second hard tube is connected to the inlet of another cut-off valve, the second hard tube is also connected to another pressure sensor connected to the industrial personal computer, the outlet of the cut-off valve is connected to the inlet of a filter, the outlet of the filter is respectively connected to the inlet of an electric needle valve and the inlet of a needle valve group, the electric signal end of the electric needle valve is connected to the industrial personal computer, and both the outlet of the electric needle valve and the outlet of the needle valve group are connected to a waste solution container. The first needle valve, the second needle valve and the third needle valve constitute the needle valve group, the first needle valve, the second needle valve and the third needle valve are arranged in series.

Further technical solutions are as follows:
The top of the reaction kettle is also connected with a rupture disk for protecting the safety of the reaction kettle through a hard tube, and the middle of the hard tube provided with the rupture disk is also wrapped with a first ice bag.
The outlet of the safety valve is connected to a waste solution container, and the middle of the hard tube provided with the safety valve is wrapped with a second ice bag.
A first cut-off valve group for adding samples is arranged between the pipeline outlet collected by each non-return valve and the inlet of a cut-off valve, and a second cut-off valve group for taking samples out is also arranged between the inlet of the filter and the outlet of another cut-off valve.
The water pump is an ultra-high-pressure and ultra-micro-flow water pump, the maximum output pressure of the water pump is 260-300 MPa, and the output flow of the water pump is continuously adjustable between 0.1-10 ml/min.
The reaction kettle is made of a titanium alloy material, and a hard seal is adopted between a cover of the reaction kettle and a kettle body.
The rupture pressure of the rupture disk is 270-300 MPa.
The setting pressure of the safety valve is 260-270 MPa.
The middle of the first hard tube is bent to form a serpentine tube, and the serpentine tube of the first hard tube is partially immersed in a first cooling water container; and the middle of the second hard tube is also bent to form a serpentine tube, and the serpentine tube of the second hard tube is partially immersed in a second cooling water container.

### Beneficial Effects of the Present Invention

### Beneficial Effects

(I) The system of the present invention has a maximum working pressure of 250 MPa and a maximum temperature of 400°C, and the system can simulate the environments of all marine hydrothermal vents in the world by means of the working ranges of the pressure and the temperature.
(II) The present invention can continuously pump a fresh nutrient solution into the reaction kettle at a flow of 0.1-10 ml/min and discharge the waste solution, and under this working condition, the present invention can work continuously for one year, thereby providing a basis for long-term growth of microorganisms, and providing a guarantee for cultivation research of microorganisms in hydrothermal environments.
(III) The pressure, temperature and flow of the system of the present invention can be continuously and automatically adjusted, and the system has the advantage of high degree of automation.
(IV) The temperature isolation between the reaction kettle and pump valves on the upstream side of the reaction kettle can be realized by adding the first cooling water container, and furthermore, the temperature isolation between the reaction kettle and valves on the downstream side of the reaction kettle can be realized by adding the second cooling water container, so that the internal medium of each pump valve is in a normal temperature state, thereby reducing the technical requirements of the pump valves and prolonging the service life.
(V) Under the ultra-micro flow of the system, the needle valve group arranged in series is used to bear the pressure drop of up to 250 MPa, which can make a single needle valve work in a suitable valve port opening region, thereby being favorable for improving the stability of pressure control of the system, reducing the jet scouring of the valve port of the needle valve, and prolonging the service life of the needle valve.

### BRIEF DESCRIPTION OF THE DRAWINGS

### Description of the Drawings

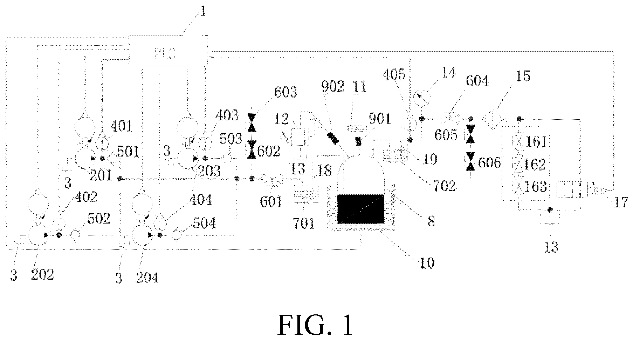

FIG. 1 is a schematic diagram of system principle of the present invention.

In the figure, 1 denotes industrial personal computer; 201 denotes first water pump; 202 denotes second water pump; 203 denotes third water pump; 204 denotes fourth water pump; 3 denotes nutrient solution container; 401 denotes first pressure sensor; 402 denotes second pressure sensor; 403 denotes third pressure sensor; 404 denotes fourth pressure sensor; 405 denotes fifth pressure sensor; 501 denotes first non-return valve; 502 denotes second non-return valve; 503 denotes third non-return valve; 504 denotes fourth non-return valve; 601 denotes first cut-off valve; 602 denotes second cut-off valve; 603 denotes third cut-off valve; 604 denotes fourth cut-off valve; 605 denotes fifth cut-off valve; 606 denotes sixth cut-off valve; 701 denotes first cooling water container; 702 denotes second cooling water container; 8 denotes reaction kettle; 901 denotes first ice bag; 902 denotes second ice bag; 10 denotes sand bath pool; 11 denotes rupture disk; 12 denotes safety valve; 13 denotes waste solution container; 14 denotes pressure gauge; 15 denotes filter; 161 denotes first needle valve; 162 denotes second needle valve; 163 denotes third needle valve; 17 denotes electric needle valve; 18 denotes first hard tube; 19 denotes second hard tube.

### DETAILED DESCRIPTION

### Embodiment of the Present Invention

As shown in FIG. 1, a deep-sea hydrothermal vent extreme environment simulation system includes an industrial personal computer 1, and motor controllers of a plurality of water pumps are connected in parallel to the industrial personal computer 1. In the present embodiment, the above-mentioned water pumps include a first water pump 201, a second water pump 202, a third water pump 203 and a fourth water pump 204. The inlets of the first water pump 201, the second water pump 202, the third water pump 203 and the fourth water pump 204 are all connected to nutrient solution containers 3, and the outlet of each water pump is respectively connected to a pressure sensor and a non-return valve. Specifically, the outlet of the first water pump 201 is respectively connected to a first pressure sensor 401 and a first non-return valve 501, the outlet of the second water pump 202 is respectively connected to a second pressure sensor 402 and a second non-return valve 502, the outlet of the third water pump 203 is respectively connected to a third pressure sensor 403 and a third non-return valve 503, and the outlet of the fourth water pump 204 is respectively connected to a fourth pressure sensor 404 and a fourth non-return valve 504. The electric signal ends of the first pressure sensor 401, the second pressure sensor 402, the third pressure sensor 403 and the fourth pressure sensor 404 above are all connected to the industrial personal computer 1. The outlets of the first non-return valve 501, the second non-return valve 502, the third non-return valve 503 and the fourth non-return valve 504 above are all collected through a pipeline. The outlet collected by the pipeline is connected to the inlet of a first cut-off valve 601, the outlet of the first cut-off valve 601 is connected to one end of a first hard tube 18, and the other end of the first hard tube 18 extends into a reaction kettle 8 and is immersed in the solution at the bottom of the reaction kettle 8. A first cut-off valve group for adding samples is also arranged between the pipeline outlet collected by each non-return valve and the inlet of the first cut-off valve 601. The first cut-off valve group includes a second cut-off valve 602 and a third cut-off valve 603 which are connected in series and are normally closed, wherein the inlet of the second cut-off valve 602 is connected to the collected pipeline, the outlet of the second cut-off valve 602 is connected to the inlet of the third cut-off valve 603, and the outlet of the third cut-off valve 603 is suspended. The middle of the first hard tube 18 is bent to form a serpentine tube, the serpentine tube of the first hard tube 18 is partially immersed in a first cooling water container 701, and the first cooling water container 701 is configured to perform temperature isolation between the reaction kettle 8 and pump valves on the upstream side of the reaction kettle 8, so that the internal medium of each pump valve can be in a normal temperature state, thereby reducing the technical requirements of the pump valves. The reaction kettle 8 is arranged inside a sand bath pool 10, an electric signal control port of the sand bath pool 10 is also connected to the industrial personal computer 1, and a temperature sensor is arranged on the sand bath pool 10.

Each of the above-mentioned water pumps is an ultra-high-pressure and ultra-micro-flow water pump, the maximum output pressure of the water pump is 260-300 MPa, the maximum output pressure of each of the above-mentioned water pumps is greater than the working pressure of the system, and the industrial personal computer 1 applies a control quantity to the motor thereof, thereby adjusting the flow of each water pump to change between 0.1-10 ml/min.

As shown in FIG. 1, the top of the reaction kettle 8 is also connected with a rupture disk 11 for protecting the safety of the reaction kettle 8 through a hard tube, and the rupture pressure of the rupture disk 11 is 270-300 MPa. The middle of the hard tube provided with the rupture disk 11 is also wrapped with a first ice bag 901. The top of the reaction kettle 8 is also connected to the inlet of a safety valve 12 through a hard tube, the outlet of the safety valve 12 is connected to a waste solution container 13, the middle of the hard tube provided with the safety valve 12 is wrapped with a second ice bag 902, and the setting pressure of the safety valve 12 is 260-270 MPa. It can be seen from the above content that the rupture pressure of the rupture disk is higher than the setting pressure of the safety valve. When the internal pressure of the reaction kettle 8 is too high, the safety valve is started to work. If the safety valve fails for some reason and the internal pressure of the reaction kettle 8 continues to rise, the rupture disk 11 ruptures and releases the pressure of the reaction kettle 8, thereby achieving the effects of protecting the reaction kettle and ensuring the safety of operators. The reaction kettle 8 is made of a titanium alloy material, and a hard seal is adopted between a cover of the reaction kettle and a kettle body. The titanium alloy material has good biocompatibility and corrosion resistance, and the hard seal mode can effectively meet the use conditions that the maximum pressure is 250 MPa and the maximum temperature is 400°C.

As shown in FIG. 1, the present invention further includes a second hard tube 19 connected to the reaction kettle 8, the second hard tube 19 is connected to the inlet of a fourth cut-off valve 604, the second hard tube 19 is also connected to a pressure gauge 14 and the inlet of a fifth pressure sensor 405, and the electric signal end of the fifth pressure sensor 405 is connected to the industrial personal computer 1. The middle of the second hard tube 19 is bent to form a serpentine tube, and the serpentine tube of the second hard tube 19 is partially immersed in a second cooling water container 702. The second cooling water container 702 is configured to perform temperature isolation between the reaction kettle 8 and valves on the downstream side of the reaction kettle 8, so that the internal medium of each valve is in a normal temperature state, thereby reducing the technical requirements of the valves. The outlet of the fourth cut-off valve 604 is connected to the inlet of a filter 15, and a second cut-off valve group for taking samples out is also arranged between the outlet of the fourth cut-off valve 604 and the inlet of the filter 15. The second cut-off valve group includes a fifth cut-off valve 605 and a sixth cut-off valve 606 which are connected in series and are normally closed, wherein the inlet of the fifth cut-off valve 605 is also connected to the outlet of the fourth cut-off valve 604, the outlet of the fifth cut-off valve 605 is connected to the inlet of the sixth cut-off valve 606, and the outlet of the sixth cut-off valve 606 is suspended. The outlet of the filter 15 is respectively connected to the inlet of an electric needle valve 17 and the inlet of a first needle valve 161, the electric signal end of the electric needle valve 17 is connected to the industrial personal computer 1, the outlet of the first needle valve 161 is connected to the inlet of a second needle valve 162, the outlet of the second needle valve 162 is connected to the inlet of a third needle valve 163, and both the outlet of the third needle valve 163 and the outlet of the electric needle valve 17 are connected to a waste solution container 13. The first needle valve 161, the second needle valve 162 and the third needle valve 163 above constitute a needle valve group. In the present invention, each of the first needle valve 161, the second needle valve 162 and the third needle valve 163 is a small-diameter needle valve with a diameter of 0.5-2.5 mm.

The control process of the target working temperature of the reaction kettle 8 is as follows:
As shown in FIG. 1, the industrial personal computer 1 controls the heating of the sand bath pool 10 to realize temperature rise, the sand bath pool 10 can heat the reaction kettle 8 to 400°C, temperature drop is realized by means of natural cooling of the reaction kettle 8, and closed-loop control of the temperature is realized by embedding a temperature sensor in the sand bath pool 10 and feeding a temperature signal back to the industrial personal computer 1, so that the working temperature of the reaction kettle 8 can be maintained at the set target temperature.

Under the flow of a nutrient solution within the system at 0.1-10 ml/min, the present invention realizes continuous work for one year through the following ways:
As shown in FIG. 1, since the first water pump 201, the second water pump 202, the third water pump 203 and the fourth water pump 204 are connected in parallel to the industrial personal computer 1, the working mode is as follows: the first water pump works normally, the second water pump is used as the first standby, the third water pump is used as the second standby, and the last water pump is used for maintenance. When the first water pump 201 is in a working state, the first water pump provides a stable target flow to the system through the first non-return valve 501. At this time, the second water pump 202 is used as the first standby, the third water pump 203 is used as the second standby, and the fourth water pump 204 is in a maintenance state. At this time, the system is under a certain steady-state pressure. Due to the existence of the second non-return valve 502, the third non-return valve 503 and the fourth non-return valve 504, the pressure of the system will not have any impact on the second water pump 202, the third water pump 203 and the fourth water pump 204. The industrial personal computer monitors the first pressure sensor 401 and the fifth pressure sensor 405 in real time. When the pressure difference between the first pressure sensor and the fifth pressure sensor is greater than 5 MPa, it is determined that the first water pump 201 is damaged and cannot provide a stable target flow. Or under the condition that although no damage to the first water pump 201 has been detected, the continuous working time of the first water pump has exceeded 2/3 of the mean working time between failures of this type of water pump, at this time, the industrial personal computer 1 starts the second water pump 202 as the first standby to provide a stable target flow. In this state, the third water pump 203 will be the first standby, the fourth water pump 204 will be the second standby, and the first water pump 201 will be in a maintenance state. The processes are circulated to ensure that the whole system can work continuously for one year.

The control of the target working pressure of the system in the present invention is realized through the following ways:
As shown in FIG. 1, when a normally working water pump (any one of the first water pump 201, the second water pump 202, the third water pump 203 and the fourth water pump 204 in a normal working state) works, this water pump provides a stable target flow for the system of the present invention. If the target flow is less than 1 ml/min, since the flow is very small, the traditional means cannot perform pressure control of the system. At this time, the first needle valve 161, the second needle valve 162 and the third needle valve 163 are closed, the industrial personal computer 1 detects the feedback value of the fifth pressure sensor 405, and the pressure is adjusted by controlling the electric needle valve 17 to open intermittently. The above design is based on the formula Δp = *Eₑ*Δ*q*Δ*T*/*V*, (wherein Δp represents a pressure change value of a dynamic closed cavity within Δ*T* time, Δ*q* represents a difference in flow between the liquids flowing in and out of the dynamic closed cavity within Δ*T* time, V represents a total volume of the dynamic closed cavity, and Eₑ represents an effective volume of elastic modulus of liquid flow). Under the condition that Δ*q* is the target flow set by the system, the present invention realizes the control of the working pressure of the system by adjusting and controlling the duration of the Δ*T* time (that is, the opening time of the electric needle valve 17).

If the set target flow is greater than 1 ml/min, a certain water pump that works normally will continue to provide a stable target flow for the system of the present invention. At this time, the electric needle valve 17 is closed, the first needle valve 161 and the second needle valve 162 are in a slightly open state and are used as fixed liquid resistance to undertake the function of partial pressure, and the opening of the third needle valve 163 is adjusted (that is, the magnitude of the variable liquid resistance is adjusted) to realize the control of the target pressure of the system. The first needle valve 161, the second needle valve 162 and the third needle valve 163 are arranged in series, each needle valve bears a certain pressure drop, and the three needle valves together bear a pressure drop of up to 250 MPa. This serial arrangement mode can make a single needle valve work in a suitable valve port opening region, thereby being favorable for improving the stability of pressure control of the system, reducing the jet scouring of the valve port of the needle valve, and prolonging the service life of the needle valve.

## Claims

1. A deep-sea hydrothermal vent extreme environment simulation system for the culture of microorganisms, comprising an industrial personal computer (1), wherein motor controllers of a plurality of water pumps are connected in parallel to the industrial personal computer (1), the inlet of each water pump is connected to a nutrient solution container (3), the outlet of each water pump is respectively connected to a pressure sensor and a non-return valve, the electric signal end of each pressure sensor is connected to the industrial personal computer (1), the outlet of each non-return valve is collected through a pipeline, the outlet collected by the pipeline is connected to the inlet of a cut-off valve, the outlet of the cut-off valve is connected to one end of a first hard tube (18), the other end of the first hard tube (18) extends into a reaction kettle (8) and is immersed at the bottom of the solution inside the reaction kettle (8), the reaction kettle (8) is arranged inside a sand bath pool (10), and an electric signal control port of the sand bath pool (10) is also connected to the industrial personal computer (1); and the system further comprises a second hard tube (19) connected to the reaction kettle (8), the second hard tube (19) is connected to the inlet of another cut-off valve, the second hard tube (19) is also connected to another pressure sensor connected to the industrial personal computer (1), the outlet of the cut-off valve is connected to the inlet of a filter (15), the outlet of the filter (15) is respectively connected to the inlet of an electric needle valve (17) and the inlet of a needle valve group, the electric signal end of the electric needle valve (17) is connected to the industrial personal computer (1), and both the outlet of the electric needle valve (17) and the outlet of the needle valve group are connected to a waste solution container (13); the first needle valve (161), the second needle valve (162) and the third needle valve (163) constitute the needle valve group, the first needle valve (161), the second needle valve (162) and the third needle valve (163) are arranged in series.

2. The deep-sea hydrothermal vent extreme environment simulation system for the culture of microorganisms according to claim 1, wherein the top of the reaction kettle (8) is also connected with a rupture disk (11) for protecting the safety of the reaction kettle (8) through a hard tube, and the middle of the hard tube provided with the rupture disk (11) is also wrapped with a first ice bag (901).

3. The deep-sea hydrothermal vent extreme environment simulation system for the culture of microorganisms according to claim 2, wherein the top of the reaction kettle (8) is also connected to the inlet of a safety valve (12) through a hard tube, the outlet of the safety valve (12) is connected to the waste solution container (13), and the middle of the hard tube provided with the safety valve (12) is wrapped with a second ice bag (902).

4. The deep-sea hydrothermal vent extreme environment simulation system for the culture of microorganisms according to claim 1, wherein a first cut-off valve group for adding samples is arranged between the pipeline outlet collected by each non-return valve and the inlet of a cut-off valve, and a second cut-off valve group for taking samples out is also arranged between the inlet of the filter and the outlet of another cut-off valve.

5. The deep-sea hydrothermal vent extreme environment simulation system for the culture of microorganisms according to claim 1, wherein the water pump is an ultra-high-pressure and ultra-micro-flow water pump, the maximum output pressure of the water pump is 260-300 MPa, and the output flow of the water pump is continuously adjustable between 0.1-10 ml/min.

6. The deep-sea hydrothermal vent extreme environment simulation system for the culture of microorganisms according to any one of claims 1-3, wherein the reaction kettle is made of a titanium alloy material, and a hard seal is adopted between a cover of the reaction kettle and a kettle body.

7. The deep-sea hydrothermal vent extreme environment simulation system for the culture of microorganisms according to claim 2, wherein the rupture pressure of the rupture disk (11) is 270-300 MPa.

8. The deep-sea hydrothermal vent extreme environment simulation system for the culture of microorganisms according to claim 3, wherein the setting pressure of the safety valve (12) is 260-270 MPa.

9. The deep-sea hydrothermal vent extreme environment simulation system for the culture of microorganisms according to claim 1, wherein the middle of the first hard tube (18) is bent to form a serpentine tube, and the serpentine tube of the first hard tube (18) is partially immersed in a first cooling water container (701); and the middle of the second hard tube (19) is also bent to form a serpentine tube, and the serpentine tube of the second hard tube (19) is partially immersed in a second cooling water container (702).

## Patentansprüche

1. Ein System zur Simulation der extremen Umgebung einer hydrothermalen Tiefseeentlüftung für die Kultur von Mikroorganismen, das einen industriellen Personal-Computer (1) beinhaltet, wobei die Motorsteuerungen einer Vielzahl von Wasserpumpen mit dem industriellen Personal-Computer (1) parallel geschaltet sind, der Einlass jeder Wasserpumpe mit einem Nährlösungsbehälter (3) verbunden ist, der Auslass jeder Wasserpumpe jeweils mit einem Drucksensor und Rückschlagventil verbunden ist, das elektrische Signalende jedes Drucksensors mit dem industriellen Personal-Computer (1) verbunden ist, der Auslass jedes Rückschlagventils durch eine Pipeline gesammelt wird, der durch die Pipeline gesammelte Auslass mit dem Einlass eines Abschaltventils verbunden ist, der Auslass des Abschaltventils mit einem Ende einer ersten Hochvakuumröhre (18) verbunden ist, das andere Ende der ersten Hochvakuumröhre (18) sich in einen Reaktionskessel (8) erstreckt und an dem Boden der Lösung innerhalb des Reaktionskessels (8) eingetaucht ist, der Reaktionskessel (8) innerhalb eines Sandbadpools (10) angeordnet ist und ein Anschluss des Sandbadpools (10) zur Steuerung eines elektrischen Signals auch mit dem industriellen Personal-Computer (1) verbunden ist; und das System ferner eine zweite Hochvakuumröhre (19) beinhaltet, die mit dem Reaktionskessel (8) verbunden ist, wobei die zweite Hochvakuumröhre (19) mit dem Einlass eines anderen Abschaltventils verbunden ist, die zweite Hochvakuumröhre (19) auch mit einem anderen Drucksensor verbunden ist, der mit dem industriellen Personal-Computer (1) verbunden ist, der Auslass des Abschaltventils mit den Einlass eines Filters (15) verbunden ist, der Auslass des Filters (15) jeweils mit dem Einlass eines elektrischen Nadelventils (17) und dem Einlass einer Nadelventilgruppe verbunden ist, das elektrische Signalende des elektrischen Nadelventils (17) mit dem industriellen Personal-Computer (1) verbunden ist und sowohl der Auslass des elektrischen Nadelventils (17) als auch der Auslass der Nadelventilgruppe mit einem Abfalllösungsbehälter (13) verbunden sind; das erste Nadelventil (161), das zweite Nadelventil (162) und das dritte Nadelventil (163) die Nadelventilgruppe darstellen, das erste Nadelventil (161), das zweite Nadelventil (162) und das dritte Nadelventil (163) in Reihe geschaltet sind.

2. System zur Simulation der extremen Umgebung einer hydrothermalen Tiefseeentlüftung für die Kultur von Mikroorganismen gemäß Anspruch 1, wobei der Oberteil des Reaktionskessels (8) durch eine Hochvakuumröhre auch mit einer Platzscheibe (11) zum Schützen der Sicherheit des Reaktionskessels (8) verbunden ist und der Mittelteil der mit der Platzscheibe (11) bereitgestellten Hochvakuumröhre auch mit einem ersten Eisbeutel (901) umwickelt ist.

3. System zur Simulation der extremen Umgebung einer hydrothermalen Tiefseeentlüftung für die Kultur von Mikroorganismen gemäß Anspruch 2, wobei der Oberteil des Reaktionskessels (8) durch eine Hochvakuumröhre auch mit dem Einlass eines Sicherheitsventils (12) verbunden ist, der Auslass des Sicherheitsventils (12) mit dem Abfalllösungsbehälter (13) verbunden ist und der Mittelteil der mit dem Sicherheitsventil (12) bereitgestellten Hochvakuumröhre mit einem zweiten Eisbeutel (902) umwickelt ist.

4. System zur Simulation der extremen Umgebung einer hydrothermalen Tiefseeentlüftung für die Kultur von Mikroorganismen gemäß Anspruch 1, wobei eine erste Abschaltventilgruppe zum Hinzufügen von Proben zwischen dem von jedem Rückschlagventil gesammelten Pipelineauslass und dem Einlass eines Abschaltventils angeordnet ist und eine zweite Abschaltventilgruppe zum Entnehmen von Proben auch zwischen dem Einlass des Filters und dem Auslass eines anderen Abschaltventils angeordnet ist.

5. System zur Simulation der extremen Umgebung einer hydrothermalen Tiefseeentlüftung für die Kultur von Mikroorganismen gemäß Anspruch 1, wobei die Wasserpumpe eine Ultrahochdruck- und Ultramikroflusswasserpumpe ist, der maximale Ausgangsdruck der Wasserpumpe 260-300 MPa beträgt und der Ausgangsfluss der Wasserpumpe zwischen 0,1-10 ml/min stufenlos einstellbar ist.

6. System zur Simulation der extremen Umgebung einer hydrothermalen Tiefseeentlüftung für die Kultur von Mikroorganismen gemäß einem der Ansprüche 1-3, wobei der Reaktionskessel aus einem Titanlegierungsmaterial hergestellt ist und zwischen einem Deckel des Reaktionskessels und einem Kesselkörper eine Hartdichtung verwendet wird.

7. System zur Simulation der extremen Umgebung einer hydrothermalen Tiefseeentlüftung für die Kultur von Mikroorganismen gemäß Anspruch 2, wobei der Brechdruck der Platzscheibe (11) 270-300 MPa beträgt.

8. System zur Simulation der extremen Umgebung einer hydrothermalen Tiefseeentlüftung für die Kultur von Mikroorganismen gemäß Anspruch 3, wobei der Setzdruck des Sicherheitsventils (12) 260-270 MPa beträgt.

9. System zur Simulation der extremen Umgebung einer hydrothermalen Tiefseeentlüftung für die Kultur von Mikroorganismen gemäß Anspruch 1, wobei der Mittelteil der ersten Hochvakuumröhre (18) geknickt ist, um eine schlangenförmige Röhre zu bilden, und die schlangenförmige Röhre der ersten Hochvakuumröhre (18) teilweise in einem ersten Kühlwasserbehälter (701) eingetaucht ist; und der Mittelteil der zweiten Hochvakuumröhre (19) auch geknickt ist, um eine schlangenförmige Röhre zu bilden, und die schlangenförmige Röhre der zweiten Hochvakuumröhre (19) teilweise in einem zweiten Kühlwasserbehälter (702) eingetaucht ist.

## Revendications

1. Un système de simulation d'environnement extrême de cheminée hydrothermale en haute mer pour la culture de micro-organismes, comprenant un ordinateur personnel industriel (1), où des commandes de moteur d'une pluralité de pompes à eau sont connectées en parallèle à l'ordinateur personnel industriel (1), l'admission de chaque pompe à eau est connectée à un conteneur de solution nutritive (3), l'évacuation de chaque pompe à eau est connectée respectivement à un capteur de pression et à une valve antiretour, l'extrémité de signal électrique de chaque capteur de pression est connectée à l'ordinateur personnel industriel (1), l'évacuation de chaque valve antiretour est collectée par le biais d'une canalisation, l'évacuation collectée par la canalisation est connectée à l'admission d'une valve de coupure, l'évacuation de la valve de coupure est connectée à une extrémité d'un premier tube rigide (18), l'autre extrémité du premier tube rigide (18) s'étend jusque dans une bouilloire à réaction (8) et est immergée au fond de la solution à l'intérieur de la bouilloire à réaction (8), la bouilloire à réaction (8) est agencée à l'intérieur d'un bassin de bain de sable (10), et un port de commande de signal électrique du bassin de bain de sable (10) est également connecté à l'ordinateur personnel industriel (1) ; et le système comprend en outre un deuxième tube rigide (19) connecté à la bouilloire à réaction (8), le deuxième tube rigide (19) est connecté à l'admission d'une autre valve de coupure, le deuxième tube rigide (19) est également connecté à un autre capteur de pression connecté à l'ordinateur personnel industriel (1), l'évacuation de la valve de coupure est connectée à l'admission d'un filtre (15), l'évacuation du filtre (15) est connectée respectivement à l'admission d'une valve à pointeau électrique (17) et à l'admission d'un groupe de valves à pointeau, l'extrémité de signal électrique de la valve à pointeau électrique (17) est connectée à l'ordinateur personnel industriel (1), et à la fois l'évacuation de la valve à pointeau électrique (17) et l'évacuation du groupe de valves à pointeau sont connectées à un conteneur de solution de déchets (13) ; la première valve à pointeau (161), la deuxième valve à pointeau (162) et la troisième valve à pointeau (163) constituent le groupe de valves à pointeau, la première valve à pointeau (161), la deuxième valve à pointeau (162) et la troisième valve à pointeau (163) sont agencées en série.

2. Le système de simulation d'environnement extrême de cheminée hydrothermale en haute mer pour la culture de micro-organismes selon la revendication 1, où le dessus de la bouilloire à réaction (8) est également connecté à un disque de rupture (11) pour protéger la sûreté de la bouilloire à réaction (8) par le biais d'un tube rigide, et le milieu du tube rigide pourvu du disque de rupture (11) est également enveloppé d'un premier sac à glace (901).

3. Le système de simulation d'environnement extrême de cheminée hydrothermale en haute mer pour la culture de micro-organismes selon la revendication 2, où le dessus de la bouilloire à réaction (8) est également connecté à l'admission d'une valve de sûreté (12) par le biais d'un tube rigide, l'évacuation de la valve de sûreté (12) est connectée au conteneur de solution de déchets (13), et le milieu du tube rigide pourvu de la valve de sûreté (12) est enveloppé d'un deuxième sac à glace (902).

4. Le système de simulation d'environnement extrême de cheminée hydrothermale en haute mer pour la culture de micro-organismes selon la revendication 1, où un premier groupe de valves de coupure pour ajouter des échantillons est agencé entre l'évacuation de canalisation collectée par chaque valve antiretour et l'admission d'une valve de coupure, et un deuxième groupe de valves de coupure pour faire sortir des échantillons est également agencé entre l'admission du filtre et l'évacuation d'une autre valve de coupure.

5. Le système de simulation d'environnement extrême de cheminée hydrothermale en haute mer pour la culture de micro-organismes selon la revendication 1, où la pompe à eau est une pompe à eau ultra-haute pression et ultra-micro-débit, la pression de sortie maximale de la pompe à eau est de 260 à 300 MPa, et le débit de sortie de la pompe à eau est continûment ajustable entre 0,1 et 10 ml/min.

6. Le système de simulation d'environnement extrême de cheminée hydrothermale en haute mer pour la culture de micro-organismes selon n'importe laquelle des revendications 1 à 3, où la bouilloire à réaction est réalisée en un matériau d'alliage de titane, et un joint d'étanchéité rigide est adopté entre un couvercle de la bouilloire à réaction et un corps de bouilloire.

7. Le système de simulation d'environnement extrême de cheminée hydrothermale en haute mer pour la culture de micro-organismes selon la revendication 2, où la pression de rupture du disque de rupture (11) est de 270 à 300 MPa.

8. Le système de simulation d'environnement extrême de cheminée hydrothermale en haute mer pour la culture de micro-organismes selon la revendication 3, où la pression de réglage de la valve de sûreté (12) est de 260 à 270 MPa.

9. Le système de simulation d'environnement extrême de cheminée hydrothermale en haute mer pour la culture de micro-organismes selon la revendication 1, où le milieu du premier tube rigide (18) est fléchi afin de former un tube serpentin, et le tube serpentin du premier tube rigide (18) est partiellement immergé dans un premier conteneur d'eau de refroidissement (701) ; et le milieu du deuxième tube rigide (19) est également fléchi afin de former un tube serpentin, et le tube serpentin du deuxième tube rigide (19) est partiellement immergé dans un deuxième conteneur d'eau de refroidissement (702).
